# EUROPEAN PATENT SPECIFICATION

(11) **EP 2 839 284 B1**
(45) Date of publication and mention of the grant of the patent: **07.08.2019**
(21) Application number: 13793390.9
(22) Date of filing: 22.05.2013
(51) Int. Cl.: G01N 33/542, G01N 33/533, G01N 33/543

(54) **A MICROPARTICLE ASSEMBLY**
MIKROPARTIKELANORDNUNG
ENSEMBLE DE MICROPARTICULES

(30) Priority: 22.05.2012 GB 201209034
(43) Date of publication of application: 25.02.2015
(73) Proprietor: National University of Singapore, Singapore 119077 (SG)
(72) Inventor: TRAU, Dieter, Singapore 119077 (SG); LEAR, Martin James, Singapore 119077 (SG); OCHS, Christopher, San Francisco California 94110 (US)
(74) Representative: Weickmann & Weickmann PartmbB
(86) International application number: PCT/SG2013/000210
(87) International publication number: WO 2013/176625

(56) References cited:
- WO-A1-00/66790
- WO-A1-02/074997
- US-A1- 2002 051 985
- US-A1- 2002 160 363
- US-A1- 2003 108 972
- US-A1- 2004 175 768
- CHEN L ET AL: "HIGHLY SENSITIVE BIOLOGICAL AND CHEMICAL SENSORS BASED ON REVERSIBLE FLUORESCENCE QUENCHING IN A CONJUGATED POLYMER", PROCEEDINGS OF THE NATIONAL ACADEMY OF SCIENCES, NATIONAL ACADEMY OF SCIENCES, US, vol. 96, no. 22, 26 October 1999 (1999-10-26), pages 12287-12292, XP002929398, ISSN: 0027-8424, DOI: 10.1073/PNAS.96.22.12287
- KUSHON STUART A ET AL: "Detection of DNA hybridization via fluorescent polymer superquenching", LANGMUIR, AMERICAN CHEMICAL SOCIETY, NEW YORK, NY; US, vol. 18, no. 20, 1 October 2002 (2002-10-01), pages 7245-7249, XP002410664, ISSN: 0743-7463, DOI: 10.1021/LA026211U
- HOREJSH, D. ET AL.: 'A molecular beacon, bead-based assay for the detection of nucleic acids by flow cytometry, art. e13' NUCLEIC ACIDS RESEARCH vol. 33, no. 2, 2005, XP002581473
- WANG, M. ET AL.: 'Fluoorescent bio/chemosensors based on silole and tetraphenylethene luminogens with aggregation-induced emission feature' JOURNAL OF MATERIALS CHEMISTRY vol. 20, 2010, pages 1858 - 1867, XP055173888

## Description

### Technical Field

The present invention generally relates to a bead construct. The present invention also relates to a microarray comprising a plurality of bead constructs and a method of manufacturing thereof. The present invention also relates to a method for identifying the presence of a target analyte in a sample and a kit for the same.

### Background

DNA and Protein Microarrays are important tools for diagnostic applications in research and clinical settings. Microarrays carrying small molecule binding partners are not yet commercially available and still in the research stage. Microarrays with small molecule binding partners are interesting as potential research tools in the future.

Bead microarrays carry microbeads which, in turn, are conjugated to biomolecules. Bead microarrays have significant advantages such as consistency, flexibility and fast kinetics. Bead microarrays are flexible because the surface chemistry of the beads can be tailored to suit the biomolecule to be conjugated (via linkers) on the bead. Potentially, any type of microarray such as drug-, metabolite-, lipid- or carbohydrate-microarrays could be manufactured by using beads. In state-of-the-art bead microarrays, the binding molecules (typically DNA types, oligonucleotides or proteins) are directly linked to the microbead with a linker. Typical linker chemistry may use biotin-streptavidin or N-hydroxysuccinimide activated functional groups to link the biomolecule to the microbead. Further, bead microarrays ensure consistent results since the dimensions of the beads can be made uniform.

Currently, bead arrays come in the form of "liquid arrays" or "planar arrays". In liquid arrays, beads remain in suspension while in planar arrays, beads are deposited onto a planar substrate material. In both approaches, typical DNA, protein, or antibody arrays are used today.

Both approaches, however, suffer from the need to optically label the analyte molecule that binds to the bead microarray. In DNA bead arrays, the DNA is labeled with a fluorophore or quantum dot, or other optical signatures. In antibody bead microarrays, typically a sandwich antibody assay is used with a fluorescently labeled detector antibody. The detector antibody can directly carry the optical label or a secondary optical labeled antibody is used. All such systems require the addition of one or more reagents and add complexity to the assay. Another Major drawback is the need for additional incubation steps, which increase the time required for conducting the assay.

US application US 2002/051985 discloses a bead construct, wherein a fluorophore molecule is bound to a bead. The fluorophore molecule comprises a polymer P and a quencher Q connected via an internal linker. The nature of this linker is such that it holds polymer P and quencher Q in close proximity so that the polymer fluorescence is quenched. Examples of suitable linkers are polyethylene, polyethylene oxides, polyamides, non-polymeric organic structures and related materials.

There is a need to provide a microparticle assembly that overcomes, or at least ameliorates, one or more of the disadvantages described above.

There is a need to provide a microarray that overcomes, or at least ameliorates, one or more of the disadvantages described above.

### Summary

According to a first aspect, there is provided a bead construct, comprising:
(a) a bead;
(b) a fluorophore molecule, comprising two fluorophore moieties connected to each other by at least one internal linker that provides conformational freedom to the two fluorophore moieties, wherein the internal linker is formed by H-bonds, salt bridges, electrostatic interaction, or at least one ionic bond, and wherein an optical signal emitted by the fluorophore changes when reducing the conformational freedom of the two fluorophore moieties to each other;
(c) a first linker molecule connected to the bead and to one of the two moieties of the fluorophore molecule;
(d) a second linker molecule connected to the other moieties of the fluorophore molecule; and
(e) a ligand molecule connected to the second linker, wherein upon binding of a target analyte molecule to the ligand molecule, the conformational freedom of the two fluorophore moieties to each other is reduced.

According to a second aspect, there is provided a microarray, comprising a plurality of bead constructs disposed thereon, wherein the bead constructs are as defined above.

According to a third aspect, there is provided a method for manufacturing a microarray, comprising:
depositing a plurality of bead constructs onto a
substrate, wherein the bead constructs are as defined above.

According to a fourth aspect, there is provided a method for identifying the presence of at least one target analyte in a sample, the method comprising:
providing a microarray as defined above;
incubating said microarray with said sample to thereby cause said at least one target analyte, if present in said sample, to bind to at least one ligand molecule, wherein the binding of said at least one target analyte reduces the conformational freedom of the two moieties of a flurophore molecule to each other, to produce a change in an optical signal; and
measuring said change in the optical signal, thereby identifying the presence of said at least one target analyte in said sample.

According to a fifth aspect, there is provided a kit for identifying the presence of a target analyte in a sample, the kit comprising:
a microarray as defined above, one or more reagents, and instructions for use.

Advantageously, a reagent is not required in order to identify the presence of the target analyte in the sample. Hence, an additional reagent incubating step is not required. This may aid in minimizing the time and steps involved when identifying the presence of a target analyte in the sample.

Advantageously, a reagent-less and expeditious method for identifying a target analyte in a sample is provided.

Advantageously, a single incubation step that does not require any added reagent (that is, reagentless) is all that is needed to identify the presence of a target analyte in the sample.

Advantageously, real time measurement of kinetic binding or reaction events between a target and the ligand can be obtained. In kinetic measurements performed using the microparticle assembly, a change in optical signature over time is recorded. Advantageously, this makes the measurement independent from any initial signal or background or unspecific signal.

### Definitions

The following words and terms used herein shall have the meaning indicated:
The terms "microarray" or "array" as used herein refers to an array of microparticles that are capable of identifying the presence of a target analyte in a sample. A plurality of "microparticle assemblies" or "bead constructs" can form a microarray. The terms "microarray" or "array" includes "liquid arrays" or "planar arrays".

The term "liquid arrays" refers to arrays in which microparticles or microbeads remain in suspension.

The term "planar arrays" refers to arrays in which microparticles or microbeads are deposited onto a planar substrate material.

The term "conformational freedom", when used in conjunction with a pair of moieties, multiple moieties or moiety ligand assemblies refers to the spatial boundaries of these moieties due to the allowed movement of the moieties. The conformational freedom of the moieties can change due to spatial motion or rotational motion or spatial position of two or more moieties to each other or spatial position of moieties ligand assemblies to each other. Due to the change in the conformational freedom, the optical signature or property of the moieties can change accordingly. The moieties can be subunits of the same molecule or at least two separate molecules.

The term "interacts" refers to any contact, reaction, hybridization or binding that occurs between a target analyte and a ligand.

The term "bead" refers to a spherical particle which can be in the micron-sized range, e.g. from 1 micron to about 1000 microns, or from 1 micron to 10 microns, or from 10 microns to 100 microns, or from 100 microns to
1000 microns, and may also be referred to as a "microbead"; or in the nano-sized range (e.g. from 1 nm to 1000 nm, or from 1 nm to 10 nm, or from 10 nm to 100 nm, or from 100 nm to 1000 nm), and may also be referred to as a "nanobead".

The term "microparticle" refers to a particle having a particle size in the micron-sized range, or from 0.1 microns to about 1000 microns. In one embodiment, when the microparticle is substantially spherical in shape, the particle size refers to the diameter of the microparticle, which is in the micron-sized range. Where the microparticle is spherical shaped, the "microparticle" is then termed as a "microbead". In another embodiment, where the microparticle does not have a spherical shape, the particle size may refer to the equivalent diameter of the particle relative to a spherical particle or may refer to a dimension (length, breadth, height or thickness) of the non-spherical particle.

The term "microparticle assembly" or "microbead assembly" or "microbead construct" as used herein refers to microparticles or microbeads that are coupled to a plurality of moieties that are capable of generating an optical signature. Typically, the plurality of moieties may be connected to the microparticle by a first linker molecule. The moieties may be connected to each other by an internal linker molecule. The moieties may be connected to or bound with a ligand that is capable of binding the target analyte. The ligand may also be individually bound to the microbead. Due to the presence of the moieties present in the microparticle assembly or microbead assembly, the microparticle assembly or microbead assembly change their optical signal or optical property upon binding of a target analyte so as to advantageously enable
the detection of the target analyte without the need of additional reagents.

The term "linker", as used herein, refers to a chemical entity linking at least two other entities together. Typically, a linker uses covalent bonding. Typically, a linker could be a single bond or is composed from a number of 1 to 10 atoms forming the backbone of the linker.

The term "internal linker" refers to a linker between the moieties. The internal linker provides conformational freedom, for example, rotational freedom to the moieties relative to each other. The conformational freedom is reduced upon binding of a target analyte to the ligand or other events involving the ligand such as ligand cleavage, hybridization events or other events in general causing a change in the molecular mass or stiffness of the moieties.

The term, "second linker" refers to a linker connecting a moiety to a ligand molecule. Typically the first and the second linker are linked to different moieties.

The term "organic polymer" as used herein, refers to a polymeric material which has repeating units of a backbone composed mainly of carbon atoms or a ring containing carbon atoms, and which also contains hydrogen. The polymeric material may also contain other elements such as, for example, sulphur, oxygen or nitrogen.

The term "moieties" refer to a part or subunit of a molecule or a distinct molecule. The molecule may be a fluorescent probe (also known as "fluorophore"), a dye (such as a quencher or reporter dye) or a quantum dot.

The term "population" as used herein refers to the total universe of microparticles.

The term "plurality of microparticles or microbeads or beads" as used herein refers to any number of microparticles; typically a larger number of microparticles.

The term "ligand" refers to any chemical agent that is chemically active or biological agent that is biologically active and which is capable of binding or reacting with a target analyte or an intermediary bound to the target analyte. The active agent may exhibit chemical activity and may include an environmental contaminant such as organic materials (for example, aliphatic hydrocarbon compounds, aromatic-containing compounds and chlorinated compounds) or inorganic materials (for example, metals and nitrates); a chemical warfare agent (for example, nerve agents such as sarin, soman, tabun and cyclosarin, blood agents such as arsines and hydrogen cyanide, or lachrymatory agents such as tear gas and pepper spray); a herbicide; a pesticide; a metabolite; a drug; lipids, carbohydrates, or a chemical catalyst. The active agent may exhibit biological activity and may be referred to herein as a "bioactive agent". Exemplary bioactive agents include proteins, oligopeptides, small organic molecules, coordination complexes, aptamers, cells, cell fragments, virus particles, antigens, polysaccharides and polynucleotides, which can be attached to or bonded to a microparticle.

The term "target analyte" or "analyte" refers to a substance to be detected that is capable of binding to the ligand or is reacting with the ligand. A target analyte may also be a substance to be detected for calibration purposes. Exemplary target analytes include, but are not limited to, nucleic acids, polynucleotides, drugs, hormones, proteins, enzymes, antibodies, carbohydrates,
receptors, bacteria, cells, virus particles, spores, and antigens.

The term "reagent" or "reagents" refer to a substance or a mixture of substances that is necessary to perform bioassays or microarrays. Typically, a reagent is a fluorescent labeled binding molecule, antibody, receptor or aptamer. Reagents can be mixed to create a mixture that can detect more than one target analyte.

The term "optical signature" or "optical property" as used herein refers to any optical signal or information which is obtained from the microparticle assembly by optical methods, such as fluorescence intensity, fluorescence spectra, optical spectroscopic, changes in absorption or emission maxima or minima, quenching, luminescence, chemo luminescence, Raman spectroscopy, UV-VIS spectroscopy or naked eye visualization. Of particular relevance to the present invention is an increase of fluorescence intensity, in particular this increase is caused by a reduced sterical freedom also refered to as "aggregation induced fluorescence".

The term "protein" as used herein may be defined as two or more covalently bonded amino acid, which includes proteins, polypeptides, oligopeptides and peptides.

The term "reagentless" refers to a bioassay or microarray process, protocol or method that does not require the addition of any reagent or solution other than the analyte solution.

The terms "amino acid" and "peptide", as used herein refer to both naturally occurring and synthetic amino acid and amino acid chains respectively.

The term "alkyl" used alone or as part of a larger moiety such as "haloalkyl", or "alkylamine" refers to a straight or branched, saturated aliphatic group having the
specified number of carbons, typically having 1 to 12 carbon atoms. More particularly, the aliphatic group may have 1 to 8, 1 to 6, or 1 to 4 carbon atoms. This term is exemplified by groups such as methyl, ethyl, n-propyl, isopropyl, n-butyl, isobutyl, tert-butyl, n-hexyl, and the like.

The term "alkylene" refers to a bivalent saturated straight-chained hydrocarbon, e.g., C₁-C₆ alkylene includes -(CH₂)₆-, -CH₂-CH-(CH₂)₃CH₃, and the like. The term "bivalent" means that the alkylene group is attached to the remainder of the molecule through two different carbon atoms.

The term "alkoxy" refers to the group -O-R where R is "alkyl", "cycloalkyl", "alkenyl", or "alkynyl". Examples of alkoxy groups include for example, methoxy, ethoxy, ethenoxy, and the like.

The term "amino" means -NH₂; while the terms "alkylamine" and "dialkylamine" mean -NHR and -NR₂, respectively, wherein R is an alkyl group.

The term "aryl" used alone or as part of a larger moiety means any aromatic or heteroaromatic group (both monocyclic and polycyclic moieties incorporating C, N, S, P, O, or not) and includes substituted phenyl, naphthyl, pyridyl, thiophenenyl, quinolinyl, triazolyl and the like.

The term "haloalkyl" includes mono, poly, and perhaloalkyl groups where the halogens are independently selected from fluorine, chlorine, bromine and iodine.

The term "cycloalkyl" used alone or as part of a larger moiety such as "cycloalkylalkyl" refers to a monocyclic or polycyclic, non-aromatic ring system of 3 to 20 carbon atoms, 3 to 12 carbon atoms, or 3 to 9 carbon atoms, which may be saturated or unsaturated. Examples of cycloalkyl groups include cyclopropyl, cyclobutyl,
cyclopentyl, cyclohexyl, cyclohexenyl, cyclohexa-1,3-dienyl, cyclooctyl, cycloheptanyl, norbornyl, adamantyl, and the like.

The term "alkenyl" refers to a straight or branched aliphatic group with at least one double bond. Typically, alkenyl groups have from 2 to 12 carbon atoms, from 2 to 8, from 2 to 6, or from 2 to 4 carbon atoms. Examples of alkenyl groups include ethenyl (-CH=CH₂), n-2-propenyl (allyl, -CH₂CH=CH₂), pentenyl, hexenyl, and the like.

The term "alkynyl" refers to a straight or branched aliphatic group having at least 1 site of alkynyl unsaturation. Typically, alkynyl groups contain 2 to 12, 2 to 8, 2 to 6 or 2 to 4 carbon atoms. Examples of alkynyl groups include ethynyl (-C≡CH), propargyl (-CH₂C≡CH), pentynyl, hexynyl, and the like.

The term "functionalized" refers to the incorporation of chemical bonds such as amide, imide, carbamate, carbonate, ether, thioether, and the like, into the linker portion by using substituted functionality such as O, N, S, P, C=O to replace C, CH, CH₂ and CH₃ groups.

The word "substantially" does not exclude "completely" e.g. a composition which is "substantially free" from Y may be completely free from Y. Where necessary, the word "substantially" may be omitted from the definition of the invention.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically
+/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and subgeneric groupings falling within the generic disclosure also form part of the disclosure. This includes the generic description of the embodiments with a proviso or negative limitation removing any subject matter from the genus, regardless of whether or not the excised material is specifically recited herein.

### Detailed Disclosure of Embodiments

Exemplary, non-limiting embodiments of a bead construct as defined in claim 1
for identifying the presence of a target analyte in a sample will now be disclosed.

The bead construct of the invention comprises a ligand selected such that, if said target analyte is present in said sample, said target analyte interacts with said ligand and causes a reduction in the conformational freedom between said fluorophore moieties that generates a change in an optical signal to identify said target analyte.

The change in the optical signal may be from no optical signal being present before interacting of the target analyte to the optical signal being present after interacting of the target analyte.

The change in the optical signal may be an increase in the level of optical signal after the interacting of the target analyte.

The change in the optical signal may be from a first optical signal being present before interacting of the target analyte to a second optical signal being present after interacting of the target analyte.

The change in the optical signal may be from an optical signal being present before interacting of the target analyte to no optical signal being present after interacting of the target analyte.

The reduction in the conformational freedom may be due to a change in the spatial distance between the moieties. The reduction in the conformational freedom may be due to a change in the orientation of the moieties.

The bead may have a particle size in the range from about 50 nm to about 500 microns, from 0.1 microns to about 500 microns, from 50 nm to about 100 microns, or from about 1 micron to about 10 microns. If
the bead is a spherical particle, the bead is then termed as a microbead. The microbead may be a capsule or hollow capsule. The bead may be a polymeric microparticle. The bead may be made from a material selected from the group consisting of organic polymers (such as but not limited to polystyrene, hydrogel, agarose, alginate), melamin, organic crystals or metallic microparticles (such as but not limited to gold, silver or iron oxide), inorganic microparticles (such as but not limited to silicon dioxide, titanium dioxide, glass or quantum dots) or mixtures thereof.

The bead is connected to one of the two moieties of the fluorophore molecule via a first linker molecule. In order to limit the rotational freedom of the moiety attached to the first linker molecule, the first linker molecule may terminate in a sterically demanding anchor group to which the moiety is attached. This approach will eliminate rotational freedom for the moiety to relax. The first linker molecule may be about 0.2 to about 500 nm long. The first linker molecule may be an organic polymer. The organic polymer may be selected from the group of polymers consisting of poly ethylene glycol (PEG), poly(vinylpyrrolidone) (PVPON), oligo (ethylene glycol), polyacrylamide, dextrane, poly-2-methyl-2-oxazoline (PMOXA), zwitterionic coatings (such as poly(carboxybetaine methacrylate)) and other synthetic polymers containing these polymers such as poly (L-lysine) (PLL)-graft-PEG, degradable or non-degradable polymers (such as poly(styrenesulfonate), polyanhydride, poly(acrylic acid), polyethyleneimine, polyglycolic acid and polycaprolactone) and dextran sulfate.

The fluorophore moieties are connected to each other by at least one internal linker that provides conformational freedom to the two fluorophore moieties of the fluorophore molecule. The fluorophore molecule emits light as a function of the conformational orientation of the two fluorophore moieties of the fluorophore molecule to each other. The optical property of the moieties may be affected by changes in the conformation and relative position of these moieties to each other. The changes in conformation are caused by changes to the conformational freedom of the molecule.

The moieties may be selected from the group consisting of a metal complex, a quantum dot, a fluorescent probe, a fluorescent protein, a reporter dye, a quencher dye, chromophore, rare earth complex, spin-probe, luminophore, N,N'-diphenyl-N,N'-bis(3-methylphenyl)-(1,1'-biphenyl)-4,4'-diamine (TPD), silole, tetraphenylethene (TPE), parts thereof and derivatives thereof.

The moieties may be subunits of one fluorophore molecule or may be a number of distinct fluorophore molecules such as two fluorophore molecules. When the moieties are a number of subunits, the subunits may be linked together by an internal linker formed by H-bonds, salt bridges, electrostatic interaction, at least one covalent bond or at least one ionic bond. Typical examples in which the moieties are subunits of one fluorescent molecule are siloles and tetraphenylethene (TPE) derivatives. The moieties may be a quantum dot metal particle pair. The quantum dot may emit considerable more light when the metal particle is in close proximity to the quantum dot due to surface plasmon resonance phenomena. The pair of moieties may also consist of the same chemical entity. The pair of moieties may consist of one fluorescent protein and one fluorophore or may be two fluorescent proteins. The two fluorescent proteins may act as bulky entities forcing the conformation into a state with a large distance between the two fluorescent proteins or the fluorescent protein and another fluorophore type. At this conformational stage, the distance is large and no interaction is observed. The pair of moieties may be a quencher-dye pair or a FRET dye pair.

The fluorophore molecule may be selected from one or more of the following classes in racemic, chiral and/or non-chiral forms: chemo- or bioluminescent molecules (such as Rhodamine, Fluorescein, Coumarin, Acridin, Bodipy, Dansyl, Dapoxy, Hoechst, Cy, merocyanines, DAPI, SYTO, Alexa Fluor dyes, Pechmann dyes, polymethines, biaryl or bi(heteroaryl) dyes, thiophenes, helicines, phenylenes, xanthenes, naphthylenes and their derivatives, isomers and/or conjugates thereof), photochromic molecules (such as spiropyrans, spirooxazines, quinones, diaryethenes, azobenzenes, and their derivatives, isomers and/or conjugates thereof), aggregation-induced emitters (e.g. tetraphenylethene, hexaphenylsilole, diphenyldibenzofulvene, siloles, and their derivatives, isomers and/or conjugates thereof), aggregation-quenched fluorophores, aggregation induced fluorescent fluorophores, black-hole quenchers paired with regular fluorophores, fluorescent proteins (GFP, RFP, YFP, CFP and constructs thereof), FRET dye pairs (e.g. mOrange2/mCherry, CFP/YFP, FITC/TRITC, etc.), metal-enhanced fluorophores (such as metal-ligand complexes based on porphyrins, bipyridyl, phenanthrolines, e.g. Ru(bpy)₂(dcbpy)(PF₆)₂), stacking-induced emission or quenching (e.g., derivatives, isomers and/or conjugates thereof of perylene, pyrene, terylene, etc.), Dexter and Exciplex quenchers, etc. The fluorophore molecule may be an aggregation-induced fluorophore, a mono- or a di-component fluorophore.

An internal linker molecule is used to connect a structural part of the molecule different to the structural part of the molecule to which the first linker molecule is connected to. The internal linker molecule provides a large degree of conformational freedom resulting in a typical fluorescence intensity. The internal linker molecule allows the moieties to have rotational freedom such that the moieties can have any rotational freedom between 0 and 360 degree.

The second linker connects the ligand to a moiety different to which the first linker is connected. The second linker molecule may be a short and/or stiff (conformationally locked or restricted) linker allowing direct transfer of the additional steric hindrance, as imposed on the microparticle assembly upon binding of analyte molecules, to the ligand. A linker of this design will directly translate any binding event into a restriction of the rotational and conformational freedom of the moieties, thus resulting in a change of the fluorescence signal and optical signature. The second linker may be formed from a single covalent bond.

The first and/or second linker molecule may be one of the following. The first and/or second linker molecule may be a covalent bond, a functionalized PEG (CH₂CH₂O)ₙ, an alkyl group (CH₂)ₙ, an alkylene group, an aryl group (e.g., substituted phenyl), an alkenyl group, an alkoxy group, a haloalkyl group, an aminoalkyl group, a cycloalkyl group, an alkenyl group, an alkynyl group, a metal complex, etc.

The first and/or second linker molecule may be an alkylene group i.e., -(CH₂)ₓ-, where x is a positive integer (for example, from 1 to about 18, more specifically between 1 and about 10, or more specifically between 2 and 8, or more specifically between 4 and 8.) Any substitutable hydrogen on a carbon atom in the linker may be substituted by halo, OH, NO₂, CN, COOH, COO(C₁-C₆alkyl), amino, (C₁-C₆)alkyl, (C₁-C₆)alkoxy, (C₁-C₆)alkylamino, or di(C₁-C₆)alkylamino, wherein the (C₁-C₆)alkyl and (C₁-C₆)alkoxy in any of the preceding groups may be optionally substituted with halogen, (C₁-C₆)alkoxy, COOH, COO(C₁-C₆alkyl), OH, NO₂ or CN. In addition, the carbon chain of the alkylene linker can be optionally substituted with one or more groups including an alkene, alkyne, phenylene, ether, thioether, amine, (C₁-C₆)alkylamino, di(C₁-C₆)alkylamino, ester, thioester, amide or carbamate. In one embodiment, the first and/or second linker molecule may be an alkylene group that is substituted with one or more amide groups. The alkylene group may be substituted with two amide groups.

The first and/or second linker molecule may be represented by the following structural formula (I) 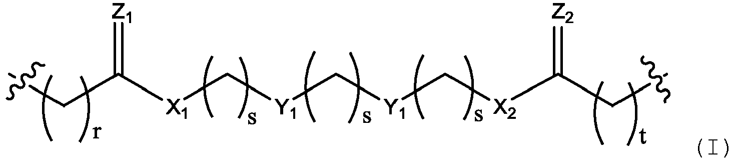 wherein each r, s, and t are independently and optionally 0, 1, 2, or 3, w is 1, and X₁, X₂, Y₁ and Y₂ are independently CR₂, O, S, or NR, wherein R is an alkyl group, an acyl group, an aryl group, a cycloalkyl group, or a heterocycle group. Alternatively, R can form an aryl group, a cycloalkyl group or a heterocycle group with another carbon atom within the linker. Z₁ and Z₂ is independently O, S or H₂.

The first and/or second linker molecule may be represented by the following structural formula (II) 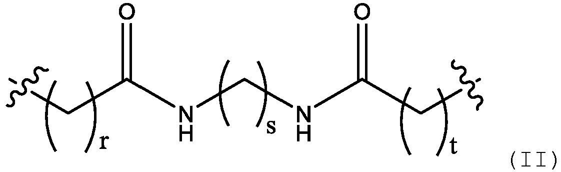 wherein r, s, and t are independently and optionally 0, 1, 2, or 3.

The first and/or second linker molecule may be represented by the following structural formula (III)

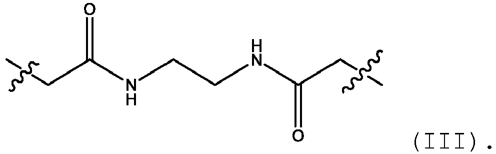

The first and/or second linker molecule may be represented by the following structural formula (IV)

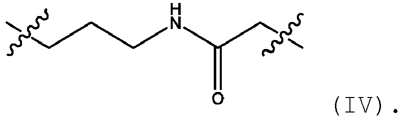

The first and/or second linker molecule may be represented by the following structural formula (V)

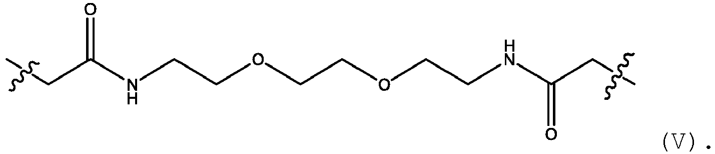

The squiggly line ( 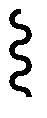 ) in the above structural formulae (I) to (V) indicates the point of connection to the moiety and the microparticle or other moieties. Either the moiety or the microparticle can be attached to either the left side or the right side of the linker molecule. Any substitutable hydrogen on a carbon atom of this linker molecule may be substituted by halo, OH, NO₂, CN, COOH, COO(C₁-C₆alkyl), amino, (C₁-C₆) alkyl, (C₁-C₆) alkoxy, (C₁-C₆)alkylamino, or di(C₁-C₆)alkylamino, wherein the (C₁-C₆) alkyl and (C₁-C₆)alkoxy in any of the preceding groups may be optionally substituted with halogen, (C₁-C₆)alkoxy, COOH, COO(C₁-C₆alkyl), OH, NO₂ or CN. Any substitutable hydrogen on a nitrogen atom may be substituted by 1 to 3 groups selected from (C₁-C₆)alkyl, optionally substituted with halogen, (C₁-C₆)alkoxy, COOH, COO(C₁-C₆alkyl), OH, NO₂ or CN. In a specific embodiment, r and t are independently 1 or 2 and s is 2 or 3.

To render the bead surface with functional groups to attach the first linker molecule, and give the microparticle antifouling properties, any form of functional coating can be applied by one of the following techniques: wet chemical deposition, chemical vapor deposition, physical vapor deposition, chemical and electrochemical techniques, spraying and variations thereof. A functional coating herein comprises coatings that can be further functionalized with the linker and/or moieties, i.e. that have some form of reactive surface functionality.

The ligand may interact with the target analyte by binding to the target analyte, by cleavage or removal of the ligand by the target analyte as well as hybridization of the target analyte. The ligand is connected to the second linker molecule. Typically, the ligand does not limit the conformational freedom, such as free rotation or other conformational changes in the moieties. Upon binding of a target analyte molecule (which is typically larger than the ligand) to the ligand, the conformational freedom (including spatial freedom or rotational freedom) is reduced, causing a direct change in fluorescence intensity and spectroscopic profile that are proportional to the binding event. By comparing and/or monitoring the optical properties before and after the addition of the sample containing target analytes, the presence of a target analyte is detected. By further determining the intensity in change of the optical property, a quantitative measurement of the analyte concentration is performed. Typically the absolute or relative changes in optical signature are first measured using standard reference materials of the analytes to obtain a calibration.

The ligand may be selected from the group consisting of a drug, a metabolite, a peptide, an antigen, a biotin, a hormone, a steroid, a toxin, an antibiotic, an allergen, a protein, a cofactor, pesticide, insecticide, oligonucleotide, polymer, biopolymer, saccharide, lipid, a metal, an electrochemically reactive ligand such as a cytochrome, parts thereof, derivatives thereof and mixtures thereof.

Methods to conjugate linker molecules with the moieties, ligands and/or microparticles can be based on all standard chemical conjugation (ligation) methods such as amide, ester, carbamate, carbonate, disulphide, maleimide, thioester, Staudinger-based, nucleophilic substitution-based (e.g., aromatic or aliphatic displacement methods), diazo-based, radical-based, carbene-based, cross metathesis-based (e.g., alkyne and alkene based) and cycloaddition-based (e.g., Huisgen 1,3-dipolar azide-alkyne 'click'-based) chemistry.

The target analyte may be an organic or an inorganic molecule. The target analyte may be selected from the group consisting of environmental pollutant (including pesticide, insecticide, toxin, etc.); a chemical (including solvent, polymer, organic material, metal ion, etc.); therapeutic molecule (including therapeutic and abused drug, antibiotic, etc.); biomolecule (including hormone, cytokine, protein, nucleic acid, lipid, carbohydrate, enzyme, antibody, antigen, cellular membrane antigen and receptor (neural, hormonal, nutrient, and cell surface receptor); whole cell (including prokaryotic (such as pathogenic bacteria) and eukaryotic cell, including mammalian tumor cell); virus (including retrovirus, herpesvirus, adenovirus, lentivirus, etc.); and spore. The target analyte may be a nucleic acid or a protein (including immunoglobulin; enzyme, hormone and cytokine). The specific binding of the target analyte to the ligand may be dependent on chemical interactions selected from the group consisting of electrostatic interaction, ionic bonds, covalent bonds, hydrogen bonds and dipole-dipole interactions.

The following design rules are in general applicable when forming a bead construct but should not be interpreted as limiting the invention. The distance between a donor and acceptor fluorophore molecule at which the energy transfer is 50% efficient is defined by the Förster radius (R₀), wherein the magnitude of R₀ depends on the spectral properties of the donor and acceptor dyes. The first fluorophore could be the donor fluorophore molecule and the second fluorophore could be the acceptor fluorophore molecule, or vice versa. The FRET efficiency (*E*) is the quantum yield of the energy transfer transition occurring per donor excitation event. The FRET efficiency depends on several parameters such as the distance between the donor and the acceptor; the spectral overlap of the donor emission spectrum and the acceptor absorption spectrum and the relative orientation of the donor and acceptor molecule. The FRET efficiency depends very strongly on the donor-to-acceptor separation distance (r), and increases with reduced distance by the 6th power law, due to the dipole-dipole coupling mechanism of FRET. At a distance of r greater than 2x R₀, almost no FRET occurs; strong FRET occurs at distances of 0.5 R₀. The design rules for the internallinker, linking the two fluorophore moieties is thus: a large distance (r) should be achieved when no binding of the target analyte onto the ligand takes place. This non-binding distance (r) is preferentially larger than 2x R₀. The distance (r) should be reduced after the binding event. This binding-event distance (r) is preferentially smaller than R₀. Because R₀ can be tuned by the selection of the donor-acceptor pair and (r) can be tuned by the linker design, optimization of R₀ and (r) will produce the most efficient bead construct.

To effect changes in the photo-physicochemical properties (i.e., optical signature changes) of the moieties, several fundamental design elements are conceivable. These comprise, but are not limited to: restriction of conformational freedom through steric, dipole-dipole, reduction of available volume or space, ionic, metal-ligand-field, oxidation, conjugation states, electrostatic, pi-stacking, aggregation and hydrogen-bonding events; pH-dependent extension or stoppage of pi-conjugation through changes in protonation or chemical states in either cyclized or linear forms; external changes in ligand-binding field effects for metal complexes through external alterations in energy quenching, energy transfer (conversion) and/or energy enhancement events.

When the bead construct, or microarray formed from the bead construct(s), are being used to analyze a sample that potentially contains a target analyte, a change in optical property is recorded as a result of the interaction between the ligand and the target analyte. In general, the change in optical property can be observed continuously and therefore referred as a kinetic measurement or, after a predetermined time, referred as an end-point measurement. The optical property of the bead construct may be detected with a detector, such as an optical detector. The optical detector may send a signal to the computer memory, which is then accessed by a computer processor for generating an image file. Data associated with the image file such as the position; type of bead and optical properties, optical spectra's and optical intensity is then used to determine an analyte or multiple analytes in a sample. The presented method enables qualitative and quantitative analysis.

Accordingly, there may be provided a microarray system for identifying the presence of one or more target analytes in a sample comprising: a microarray comprising a plurality of bead constructs, each carrying a different ligand for multiplex bioassays; a detector to detect optical properties originating from the bead constructs upon interaction between the target analyte molecules and the respective ligands of the bead constructs; and a processor responsive to program instructions to receive data from the detector to identify the presence of the one or more target analytes in the sample.

The disclosed process may enable bead constructs with high multiplexing capabilities to be generated.

In the following, different types of bioassays that can be performed using the bead constructs are disclosed. In general, any biological interaction of the ligand that causes a change in molecular weight or stiffness can be detected by the bead construct. The following types of bioassays are described in the following: a) binding assays, b) enzymatic assays based on cleavage, c) enzymatic assays based on polymerization.

In one embodiment, the bead construct is used to perform binding assays. In this assay format, a target analyte molecule binds to the ligand of the bead construct and increase the molecular weight and/or stiffness. Both events will cause a reduction in
conformational freedom resulting in a change in optical signature.

In another example, the ligand may be a single strand DNA molecule and may act as a template. Upon contact with a primer, DNTPs and a polymerase, a complementary DNA strand is formed on the ligand causing an increase in molecular weight and stiffness. Both events will cause a reduction in conformational freedom resulting in a change in optical signature. In general, enzymatic assays for hydrolases, synthetases, oxidoreductases, topomerases, transferases, lyases, isomerases and ligases can be designed as long as the molecular weight, conformational change or change in stiffness is caused by the enzyme on the ligand.

The microarray may be used for screening a number of samples. First, several batches of microparticle assemblies carrying different low molecular drug molecules as ligands are prepared. Second, a planar bead microarray is formed; encoding of the bead constructs can be done by existing methods such as spatial encoding technology. Third, the planar bead array is observed by a detector to detect changes in optical property; typically this is a change in fluorescent intensity. By using this screening technology, array biomolecules with a binding affinity to any of the present and known ligands can be detected. No reagent or additional labeling step is necessary; keeping the sample molecules in its native state. A change in optical property is directly observed upon sample addition and binding to ligands. Optional sample molecules can than be sequentially washed off and collected for further studies by the addition of the respective ligand. In this way, a high throughput screening of potential drug targets combined with the isolation is performed.

There is also provided a microarray for identifying the presence of at least one target analyte in a sample, the microarray comprising a plurality of bead constructs disposed therein, each bead construct being as defined above.

Each bead construct may carry a ligand that is different from each other so as to identify the presence of more than one target analytes in the sample.

There is also provided a method for manufacturing a microarray to identify the presence of a target analyte in a sample, comprising the step of:
depositing a plurality of bead constructs onto the microarray, each bead construct being as defined above.

There is further provided a method for manufacturing a microarray, comprising: depositing a plurality of bead constructs onto a substrate, wherein the bead constructs comprise:
(a) a bead;
(b) a fluorophore molecule, comprising two fluorophore moieties connected to each other by at least one internal linker that provides conformational freedom to the two fluorophore moieties, wherein the internal linker is formed by H-bonds, salt bridges, electrostatic interaction, or at least one ionic bond, and wherein an optical signal emitted by the fluorophore changes when reducing the conformational freedom of the two fluorophore moieties to each other;
(c) a first linker molecule connected to the bead and to one of the two moieties of the fluorophore molecule;
(d) a second linker molecule connected to the other moieties of the fluorophore molecule; and
(e) a ligand molecule connected to the second linker, wherein upon binding of a target analyte molecule to the ligand molecule, the conformational freedom of the two fluorophore moieties to each other is reduced.

The substrate is any material which is capable of supporting the beads of the present invention thereon and may include: polymeric materials, organic materials, inorganic materials, metals, ceramics, plastic, rubber, glass, fibrous materials, graphite or silicon, silicon dioxide, silicon nitride, modified silicon, glass, modified or functionalized glass, inorganic glass, plastics, acrylics, polystyrene, copolymers of styrene, polypropylene, polyethylene, polybutylene, polyurethane, Teflon, polysaccharide, nylon, nitrocellulose, resins, silica, silica-based materials and carbon.

There is also provided a method for identifying the presence of at least one target analyte in a sample, the method comprising the steps of:
providing a microarray as described above;
incubating the microarray with the sample to thereby cause at least one target analyte, if present in the sample, to bind to at least one ligand molecule, wherein the binding of at least one target analyte reduces the conformational freedom of the two moieties of a fluorophore molecule to each other to produce a change in an optical signal; and
measuring the change in the optical signal to thereby identify the presence of at least one target analyte in the sample.

There is also provided a kit for identifying the presence of a target analyte in a sample, the kit comprising a microarray as defined above; one or more reagents and instructions for use.

### Brief Description Of Drawings

The accompanying drawings illustrate the disclosed embodiments and serve to explain the principles of the
disclosed embodiments. It is to be understood, however, that the drawings are designed for purposes of illustration only, and not as a definition of the limits of the invention.
Figures 1A to 1C are schematic diagrams showing possible embodiments of the microbead construct of the present invention.
Figure 2 is a schematic diagram illustrating one of the design principles of the present invention in relation to the distance between two flurophores of a microbead construct of the present invention.
Figures 3A to 3E are schematic diagrams illustrating various exemplary bead bioassays involving the use of a microbead construct of the present invention.
Figure 4A is a graph showing the absorbance-matched emission spectra in DMSO, demonstrating an increase in fluorescence with a restriction in rotational freedom upon binding of an analyte to a microbead construct of the present invention.
Figure 4B and 4C are graphs showing the modes of detection for changes in optical signature upon binding of an analyte to a microbead construct of the present invention.

### Detailed Description of Drawings

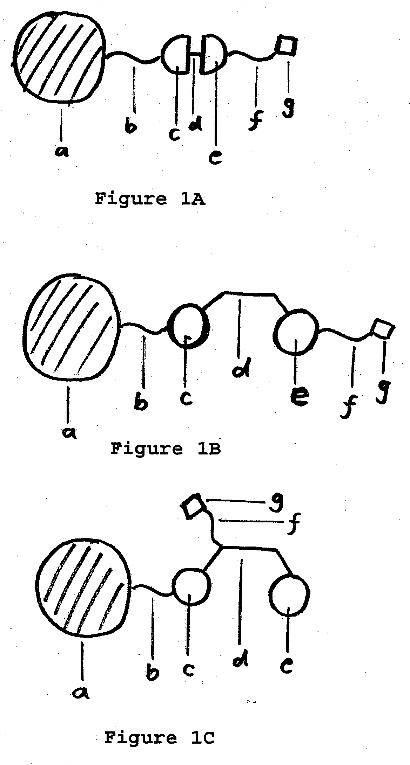

Figure 1A depicts a microbead construct of the present invention, wherein changes in the conformation and relative position of at least two parts or subunits of a single fluorophore molecule to each other result in a change in the optical signature obtained (only two subunits shown in Figure 1A for simplicity; a= microbead, b= first linker, c= first subunit (or moiety) of fluorophore, d= internal linker, e= second subunit (or moiety) of fluorophore, f= second linker, g= ligand).

In Figure 1A, upon binding of a typical high molecular-weight analyte molecule to the ligand, the conformational freedom is restricted. This results in a change in conformation and a different characteristic optical signature is obtained compared to when no analyte molecule is bound to the ligand.

The microbead constructs shown in Figures 1B and 1C illustrate another two possibleconstructs, wherein each microbead construct has two fluorophores instead of a single fluorophore(a= microbead, b= first linker, c= first fluorophore, d= internal linker, e= second fluorophore, f= second linker, g= ligand). However, the two fluorophore microbead constructs function in a similar fashion to the single fluorophore microbead construct, i.e. upon binding of an analyte molecule, a reduction in steric or conformational freedom is caused, which results in a change in the optical signature obtained.

The primary difference between the microbead constructs of Figures 1B and 1C is that the second linker of the microbead construct is attached to the second fluorophore in Figure 1B, whereas the second linker is attached to the internal linker in Figure 1C (not in accordance with the invention). Nonetheless, in both microbead constructs, the rotational freedom is via the internal linker and the two fluorophore molecules can have any rotational freedom between 0 to 360 degrees. Upon binding of a high molecular weight analyte, the rotational freedom of the microbead constructs is substantially restricted, which results in a change in the optical signature obtained.

One of the principles that should be applied to the design of a two fluorophore microbead construct (as exemplified in Figures 1B and 1C) is that the donor-to-acceptor distance between the two fluorophores should be large when the target analyte is not bound to the ligand of the microbead construct, and should be subsequently reduced after the target analyte is bound. The donor-acceptor distance should thus preferentially be twice that of the Förster radius (R₀) before the binding event, and smaller than R₀ after the target analyte is bound to the ligand of the microbead construct.

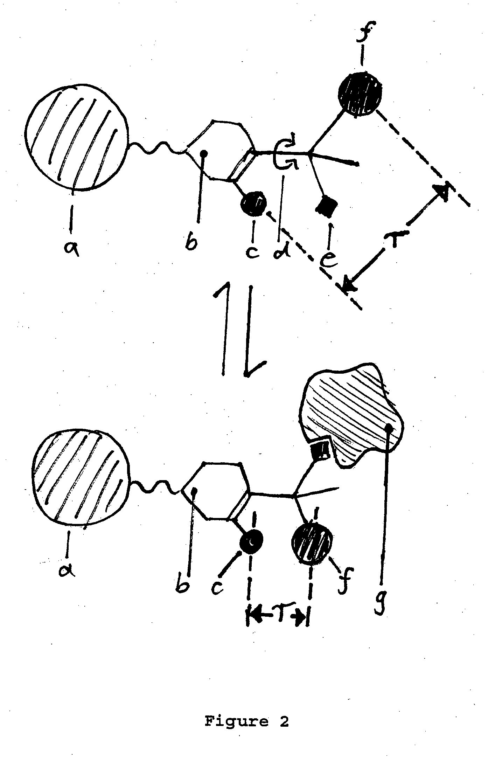

Figure 2 (not in accordance with the invention) is a schematic diagram which illustrates the above design principle [a= microbead, b= rigid part of the first linker, c= first fluorophore (or quencher, or metal particle or first subunit of a single fluorophore), d= rotational free part of the inner linker, e= ligand, f= second fluorophore (or quencher, or metal linker or second subunit of a single fluorophore), r= distance between the two fluorophores or fluorophore quencher pair or subunit pair, arrow= axis of rotational freedom].

In Figure 2, the two fluorophores (or subunits) "c" and "f" are bulky and therefore at the state with lowest energy, the distance between the fluorophores (or subunits) "r" is large, resulting in no fluorescence (above). The analyte binding onto the ligand creates an even bulkier group and is forced away from the first fluorophore "c". At the same time, this brings the second fluorophore "f" much closer to the first fluorophore "c" (much smaller "r" bellow R₀) causing fluorescence or quenching (depicted below). In this example, the ligand can also be directly linked to the second fluorophore or subunit. This variant is in accordance with the invention. It is preferable if the signal difference (for example, the fluorescence intensity) is large between the non-binding and the binding-event.

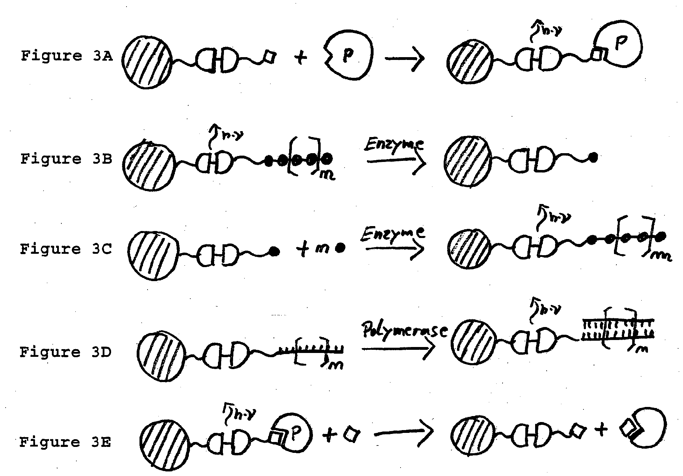

Figure 3A is a schematic diagram which shows a bead bioassay involving the use of a microbead construct of the present invention, wherein the binding of a protein or other high molecular weight molecule to the ligand of the construct results in an increase in molecular weight and/or stiffness (P= protein or other high molecular weight molecule). This in turn causes a reduction in conformation freedom and a change in the optical signature obtained.

Figure 3B (not in accordance with the invention) is a schematic diagram which illustrates a bead bioassay involving the use of a microbead construct, wherein the ligand is a protein or DNA or other polymer and a reduction in molecular weight and/or stiffness is caused by the cleavage of the protein by an analyte protease or peptidase, or cleavage of the DNA by an analyte DNAse (m = number of monomers). This in turn causes an increase in conformation freedom and a change in the optical signature obtained.

Alternatively, the ligand can also be a polyester or a polyamide or a polysaccharide, wherein a reduction in molecular weight and/or stiffness is caused by the hydrolysis of the polymer. This in turn causes an increase in conformation freedom and a change in the optical signature obtained.

Figure 3C is a schematic diagram which illustrates a bead bioassay using a microbead construct of the present invention, wherein the ligand is a monomeric subunit or a small polymer and an increase of molecular weight is caused by the extension of the ligand.

Figure 3D is a schematic diagram which illustrates a bead bioassay using a microbead construct of the present invention, wherein the ligand is a single stranded nucleic acid such as DNA or RNA and an increase in molecular weight and/or stiffness is caused by the formation of a complementary nucleic acid strand onto the ligand strand (m= number of monomers). This in turn causes a reduction in conformation freedom and a change in the optical signature obtained.

Figure 3E is a schematic diagram which illustrates a bead bioassay using a microbead construct of the present invention using a mechanism opposite to Figure 3A, wherein a large molecule bound to the ligand is displaced by the analyte reducing the molecular weight.

In all of the above examples of the bead bioassays, the optical signature may be continuously monitored.

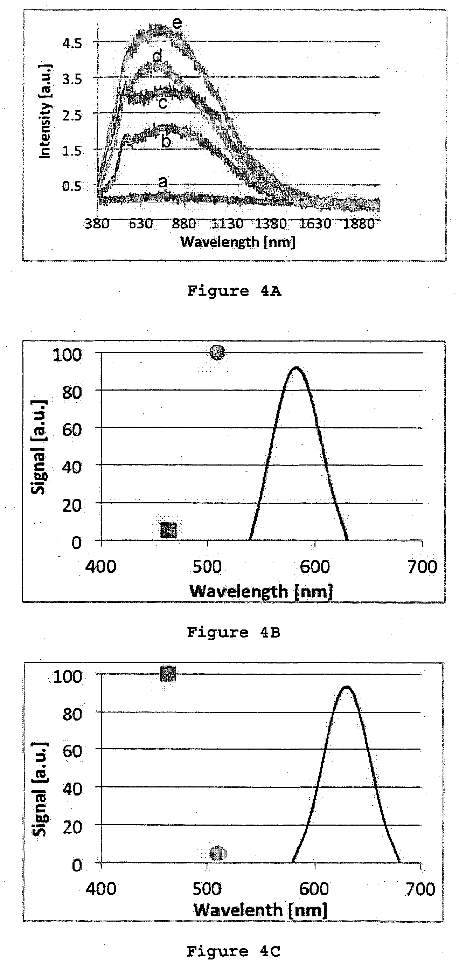

Figure 4A is a graph which shows the absorbance-matched emission spectra in DMSO, thereby demonstrating an increase in fluorescence with a restriction in rotational freedom upon binding of an analyte to a microbead construct of the present invention. a= pure DMSO, b= tetraphenylethene bisazide (TPE bisazide), c= doubleclicked TPE with alkyne-functionalized Biotin, d= monoclicked TPE with alkyne-functionalized Biotin (in which a biotin molecule is attached (clicked) onto the fluorescent molecule TPE), e= double-clicked biotin modified TPE after streptavidin binding (in which two biotin molecules are clicked onto the fluorescent molecule TPE). The attachment (or clicking) of the biotin molecule(s) onto the fluorescent molecule TPE occurs due to a method called "click chemistry", in which a chemical bond is formed between two chemical entities, one with an alkyne group and the other with an azide group. The chemical reaction is called an "Azide-Alkyne cycloaddition".

Figure 4B and 4C are graphs which show the modes of detection for changes in optical signature upon binding of an analyte to a microbead construct of the present invention. Square series= increase in signal upon binding tetraphenylethenes (TPE)/Black Hole Quencher (BHQ) dye pairs, Circle series = signal reduction using the FRET phenomenon. Line = spectral shift of Seminaphtharhodafluor (SNARF) dye.

### Examples

Non-limiting examples of the invention and a comparative example will be further described in greater detail by reference to specific Examples, which should not be construed as in any way limiting the scope of the invention.

### Example 1

This example demonstrates the synthesis of a bead construct.
**Microbead preparation:** Carboxylated silica beads (5.64 µm ± 0.27 µm) were purchased from Microparticles GmbH (Berlin, Germany) and used as received. These beads may be substituted with particulates of any size (within the range indicated above) and material (such as listed earlier), either commercially available or synthetic (home-made).
**Linker chemistry:** For this example, propargylamine was attached to the surface of EDC/NHS-ativated carboxylated silica beads. 200 microliter of bead stock solution was incubated with 1 mg/mL of EDC and 1 mg/mL NHS in PBS buffer (pH 6, Vivantis) for 2 h. After washing the beads by sequential centrifugation/redispersion cycles (3x), beads were dispersed in a solution of Propargylamine (2 mg/mL) in PBS (pH 8) for 2 h. Finally, non-reacted Alkyne was washed out by centrifugation and redispersion of the beads in DMSO (for further reactions) or water (for storage).
**Fluorophore:** As an example, a bi-functional click linker tetraphenylethene (TPE) was synthesized according to a literature protocol (Adv. Funct. Mater. 2009, 19, 1891-1900)). The bisazide click linker was then attached to the beads by exposing them to a 2 mg/mL TPE in DMSO in the presence of 2 mg/mL Copper Sulfate and 5 mg/mL Sodium Ascorbate in minute quantities of water using click chemistry.
**Ligand:** The specific interaction between biotin and streptavidin was used to demonstrate analyte binding as a proof of concept. Alkyne-functionalized Biotin acid was attached to the fluorophore via click chemistry. This facile step of pre-functionalizing the ligand with a click group allows for the attachment of second linker (short alkyl chain) and ligand (biotin) in one step.

### Example 2

This example demonstrates a binding assay using the bead construct and a conventional fluorescent spectrometer or fluorescent reader. Beads of the current invention were prepared comprising a ligand that can undergo a binding event for the detection of an analytes and were dispersed in 0.01 M PBS buffer. An aliquot of the bead suspension was pipetted into a cuvette and the fluorescence intensity was measured (baseline). An aliquot of the target analyte sample solution was added and the change in fluorescence intensity was continuously measured or recorded after 1 minute (measurement). A control measurement of the sample solution for intrinsic fluorescence was performed. A calibration curve was generated by repeating the above protocol with standard reference materials of the analyte. The analyte concentration of the sample was determined by comparing the change in fluorescence intensity of the sample with the calibration curve.

### Example 3

This example demonstrates multiplex detection by forming a bead microarray from sub-batches of the bead construct.
**Bead assembly on planar substrate:** Several batches of beads of the current invention were prepared, comprising different ligands for the detection of several different analytes and are dispersed in 0.01 M PBS buffer. A bead microarray was formed by assembling the beads in a random fashion on a planar substrate as taught in PCT/SG2007/000232 "A Microarray System and a Process for Producing Microarrays". By applying several batches sequentially a microarray carrying sub-populations of beads was formed. Beads were identified by their spatial position on the microarray.
**Bioassay protocol:** An aliquot of the target analyte sample solution was added and incubated for 3 to 30 minutes. After incubation the array was washed with a washing buffer, e.g. 0.1 M PBS.
**Optical imaging:** The array was placed under a fluorescent microscope and the fluorescence intensity is measured (end point method). Alternative changes in fluorescent intensity are measured continuously (kinetic method). A calibration curve was generated by repeating the above protocol with all standard reference materials of the analytes. The analyte concentration of the sample was determined by comparing the change in fluorescence intensity of the sample with the calibration curve.

### Example 4

The following is a non-limiting example for the synthesis of compounds exhibiting aggregation induced fluorescence with functional groups on two moieties of the compound to attach linkers is given. Compound 8 is an example carrying a first and a second linker for coupling to a microbead and a ligand respectively.

### Synthesis and Chemical Analysis of the Fluorophores Amine TPE

4-Aminebenzophenone (1, 400 mg, 2.03 mmol) was dissolved in THF (15 ml). The solution was cooled to 0°C and Zn dust (332 mg, 5.07 mmol, 2.5 equiv.) and TiCl₄ (1 M in toluene, 4.06 ml, 4,06 mmol, 2 equiv.) were added. After refluxing for 4 h, 50 ml of a 10 % K₂O₃ solution was added and stirred overnight at room temperature. The white precipitate (2) was extracted 3 x with DCM.

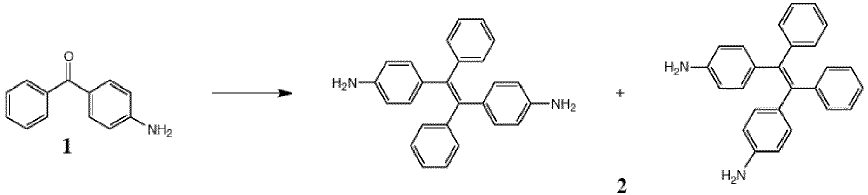

### Analysis of 2:

**1H-NMR** (400 MHz, CDCl₃): δ (ppm) = 7.26-7.06 (m, 10 H, Harom), 6.67-6.76 (m, 4 H, Harom),6.41-6.39 (m, 4 H, Harom), 3.54 (s, 4 H, Hamine)

### Carboxyl TPE

4-Methylbenzophenone (3, 2.0 g, 10.2 mmol, 1.0 equiv.) was dissolved in THF (25.5 ml) under ni- trogen. The solution was cooled to -78 °C, and TiCl₄ (1 M in toluene, 12 ml, 12.0 mmol, 1.2 equiv.) and Zn dust (1.33 g, 20.4 mmol, 2.0 equiv.) were added. After refluxing overnight, the reaction mixture was cooled to room temperature and filtered through a pad of silica gel. The filtrate was concentrated and the crude product was purified by flash chromatography (elution: hexane/ethyl acetate = 98/2). The desired compound 4 was obtained as an off white solid in 80 % yield (2.93 g). Analytical data matched the published ones.

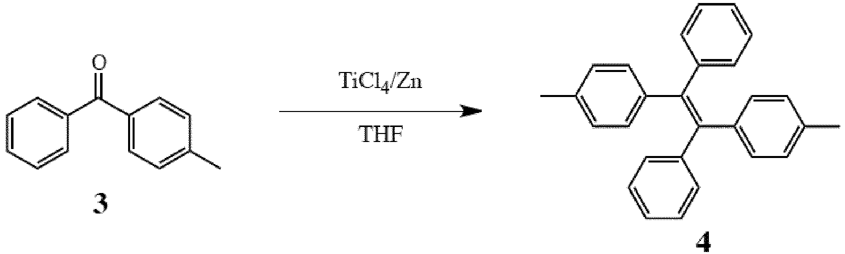

**4** (1.17 g, 3.25 mmol, 1.0 equiv.), freshly recrystallized NBS (1.45 g, 8.12 mmol, 2.5 equiv.) and benzoyl peroxide (157 mg, 0.65 mmol, 0.2 equiv.) was dissolved in 108 ml of carbon tetrachloride. The solution was refluxed for 20 h. After cooled to room temperature, the solution was filtered and the filtrate was concentrated. The crude product was purified by flash chromatography (elution: hexane/DCM = 6/4). The desired compound 5 was obtained as yellow oil in 50 % yield (850 mg). Analytical data matched the published ones.

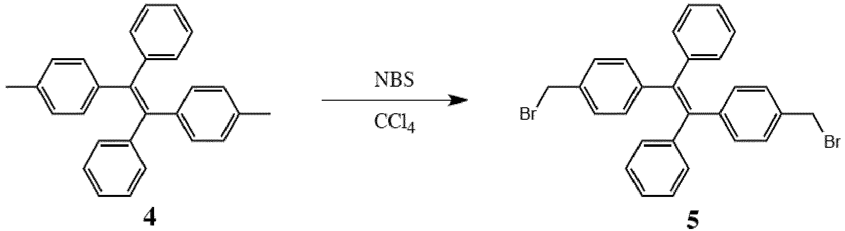

Dibromo compound 5 (850 mg, 1.64 mmol, 1.0 equiv.) and sodium azide (267 mg, 4.10 mmol,2.5 equiv.) were dissolved in 41 ml of DMSO. After stirring at room temperature for 2 days, small amounts of water were added to quench the reaction, during which the solution temperature was increased slightly. After the solution cooled to room temperature, the solution was extracted with diethyl ether five times. The organic phases were combined, washed with water and brine, and then dried over MgSO₄. After filtration and solvent evaporation, the crude product was purified by flash chromatography (elution: hexane/DCM = 9/1 to 7/3). The desired compound 6 was obtained as yellow oil in 84 % yield (615 mg).

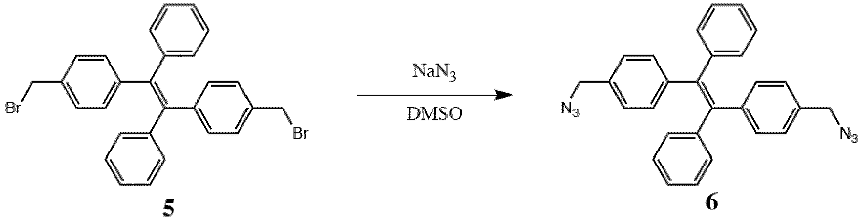

Diazide compound 6 (190 mg, 0.43 mmol, 1.0 equiv.) and 4-pentynoic acid (84 mg, 0.86 mmol, 2.0 equiv.) were dissolved in 35.8 ml of t-BuOH and 7.2 ml of H₂O. Freshly prepared aqueous solutions of CuSO₄ (86 fll, c = 1 M, 0.086 mmol, 0.2 equiv.) and sodium ascorbate (86 fll, c = 1 M, 0.086 mmol, 0.2 equiv.) were added every few hours, until a total amount of 1 equiv. was reached for both of them. After stirring at room temperature for a total of 24 h, the starting material was still detected in the TLC. The reaction was nevertheless quenched with brine to avoid a conversion to the bi-click product only. The solution was extracted diethyl ether five times; the organic phases were combined, washed with brine, and then dried over MgSO₄. After filtration and solvent evaporation, the crude product was purified by flash chromatography (elution: DCM/MeOH =95/5 to 8/2). Compound 7 was obtained as yellow oil in 28 % yield (66 mg), along with the bi- click product 8 as yellow solid in 10 % yield (28 mg), and some starting material (41 mg, 21 %).

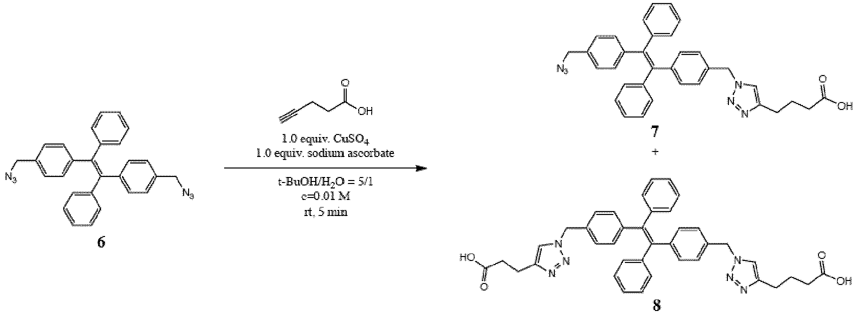

### Analysis of 7:

**1H-NMR** (400 MHz, CDCl₃): δ (ppm) = 7.21-6.96 (m, 19 H, Harom and Htriazole), 5.37 (s, 2 H, Ph-CH₂-Ntriazole), 4.32 (s, 2 H, Ph-CH2-N3), 3.00 (m, 2 H, CH2), 2.75 (m, 2 H, CH2)
**13C-NMR** (400 MHz, CDCl3): δ (ppm) = 177.3 (C4, COOH), 146.6 (C4, Carom), 144.3 (C4, Ctriazole), 143.7 (C4, Carom), 143.2 (C4, Carom), 141.2 (C4, Carom), 140.7 (C4, Carom), 133.6 (C4, C=C), 132.8 (C4, C=C), 132.0 (C3, Carom), 131.8 (C3, Carom), 131.3 (C3, Carom), 128.0 (C3, Carom), 127.8 (C3, Carom), 127.5 (C3, Carom), 126.8 (C3, arom), 121.4 (C3, Ctriazol), 54.6 (C2, Ph-CH2-N3), 54.0 (C2, Ph-CH2-triazole), 20.9 (C2, CH2-COOH)
**IR (KBr):** v (cm-1) = 3414 (COOH), 2923, 2096 (N3), 1690 (COOH), 1443, 1261, 1205, 1062,797, 697
In general, the synthesis of fluorophores containing two linkers for conjugation to a microbead and a ligand can be performed with other classes of fluorophores such as: arylenevinylene systems (e.g. cis 2,5-diphenyl-1,4-distyrylbenzene J Phys Chem B. 2006 Oct 26;110 (42) :20993-1000, DOI:10.1021/jp064069q) triphenylamine-based diketo-pyrrolo-pyrroles (Australian Journal of Chemistry 65(4) 387-394, http://dx.doi.org/10.1071/CH11410) Cholesteryl substituted pyrane derivatives (J. Phys. Chem. B, 2007, 111 (8), pp 2000-2007 / DOI:10.1021/jp067374k) 8,8a-dihydrocyclopenta[a]indene-derivatives (Angewandte Chemie International Edition, 2008, 47, 51, 9891-9894, DOI: 10.1002/anie.200802560) Salicylaldehyde Azines (e.g. N,N'-disalicylalacine: J. Org. Chem., 2009, 74 (5), pp 2163-2166, DOI:10.1021/jo802631m) p-phenyleneethylnylene substituted o-carboranes (Macromolecules, 2009, 42 (5), pp 1418-1420 / DOI: 10.1021/ma8027358) poly-triazole polymers (Macromolecules, 2009, 42 (5), pp 1421-1424, DOI: 10.1021/ma8024706), polyphenylenes polyarylenes triphenylamine-fluorenones triphenylamine-benzaldehydes dendritic phosphole-sulphides diphenylfumaronitrile derivatives benzene-1,3,5-tricarboxamide derivatives poly(1-phenyl-1-alkyne)s poly(diphenylacetylene)s, 11,11,12,12,-tetracyano-9,10-anthraquinodimethane, tetraphenylethene derivative, 2,3,4,5-tetraphenylcyclopenta-2,4-dienyl benzene derivatives, compounds containing triphenylamine-anthrylenevinylene and tetraphenylethene moieties or tetraphenylthiophene moiety, 4,4'-bis(1,2,2-triphenylvinyl)biphenyl, triarylcyclopentadiene compounds, dendritic phosphole oxides, 1,8-naphthalimide based compounds, trifluoromethyl substituted distyrylbenzenes, naphthalimide derivatives, diphenyldibenzofulvene, poly(pyridinium salt)s, tetranaphthylethene compounds, benzobis(thiadiazole)-based compounds.

### Applications

Advantageously, the disclosed method provides a generic method for binding assays on a microarray format.

More advantageously, the disclosed method may allow the formation of large libraries of microparticle assemblies carrying a large variety of ligands for pharmaceutical screening applications.

Hence, the disclosed method may overcome problems associated with the prior art, such as changes in analyte binding affinity and characteristics due to labels, multi step assay protocols, non-compatibility of different assays, limits in multiplexing, limitations in kinetic analysis and long assay times.

Advantageously, the disclosed method provides an improved method to perform bioassays, in particular multiplex bioassays on a planar bead microarray format.

Advantageously, real time measurement of kinetic binding or reaction events between a target and the ligand can be obtained. In kinetic measurements performed using the microparticle assembly, a change in optical signature over time is recorded. Advantageously, this makes the measurement independent from any initial signal or background or unspecific signal.

The disclosed method may also be combined with state-of-the-art microarray methods to improve the performance of such known methods substantially.

## Claims

1. A bead construct, comprising:
(a) a bead;
(b) a fluorophore molecule, comprising two fluorophore moieties connected to each other by at least one internal linker that provides conformational freedom to the two fluorophore moieties, wherein the internal linker is formed by H-bonds, salt bridges, electrostatic interaction, or at least one ionic bond, and wherein an optical signal emitted by the fluorophore changes when reducing the conformational freedom of the two fluorophore moieties to each other;
(c) a first linker molecule connected to the bead and to one of the two moieties of the fluorophore molecule;
(d) a second linker molecule connected to the other moieties of the fluorophore molecule; and
(e) a ligand molecule connected to the second linker, wherein upon binding of a target analyte molecule to the ligand molecule, the conformational freedom of the two fluorophore moieties to each other is reduced.

2. The bead construct according to claim 1, wherein said fluorophore molecule is selected from the group consisting of: chemo- or bioluminescent molecules, aggregation-quenched fluorophores, aggregation induced fluorescent fluorophores, black-hole quenchers paired with regular fluorophores, fluorescent proteins, FRET dye pairs, metal-enhanced fluorophores, stacking-induced emission or quenching fluorophores, Dexter and Exciplex quenchers; and optionally wherein the fluorescent protein is selected from the group consisting of: GFP, RFP, YFP, CFP and constructs thereof; and optionally
wherein the FRET dye pairs are selected from the group consisting of: mOrange2/mCherry, CFP/YFP and FITC/TRITC; and optionally
wherein the chemo or bioluminescent molecule is selected from the group consisting of: Rhodamine, Fluorescein, Coumarin, Acridin, Bodipy, Dansyl, Dapoxy, Hoechst, Cy, merocyanines, DAPI, SYTO, Alexa Fluor dyes, Pechmann dyes, polymethines, biaryl or bi(heteroaryl) dyes, thiophenes, helicines, phenylenes, xanthenes, naphthylenes and their derivatives, isomers and conjugates thereof, photochromic molecules and aggregation-induced emitters; and optionally
wherein the photochromic molecules are selected from the group consisting of: spiropyrans, spirooxazines, quinones, diaryethenes, azobenzenes, and their derivatives, isomers and conjugates thereof; and optionally
wherein the aggregation-induced emitters are selected from the group consisting of: tetraphenylethene, hexaphenylsilole, diphenyldibenzofulvene, siloles and their derivatives, isomers and conjugates thereof; and optionally
wherein the metal-enhanced fluorophores are selected from the group consisting of: metal-ligand complexes based on porphyrins, bipyridyl and phenanthrolines; and optionally
wherein the stacking-induced emission or quenching fluorophores are selected from the group consisting of: perylene, pyrene, terylene and their derivatives, isomers and conjugates thereof.

3. The bead construct according to claim 2, wherein the fluorophore molecule is an aggregation-induced fluorophore, or a mono-component, or a di-component fluorophore.

4. The bead construct according to any one of the preceding claims, wherein the bead has a diameter from 50 nm to 100 micrometer; and optionally wherein said first linker molecule has a length in the range of 0.2 nm to 500 nm.

5. The bead construct according to any one of the preceding claims, wherein said first linker molecule comprises an organic polymer; and optionally wherein said organic polymer is selected from the group consisting of polyethylene glycol, poly(vinylpyrrolidone), oligo(ethylene glycol), polyacrylamide, dextran, poly-2-methyl-2-oxazoline, poly(carboxybetaine methacrylate), poly (L-lysine), poly(styrenesulfonate), polyanhydride, poly(acrylic acid), polyethyleneimine, polyglycolic acid and polycaprolactone.

6. The bead construct according to any one of the preceding claims, wherein said second linker molecule has a length in the range of 0.1 nm to 10 nm.

7. The bead construct according to any one of the preceding claims, wherein said first and/or second linker molecule is selected from the group consisting of: 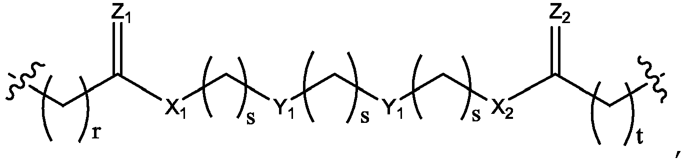 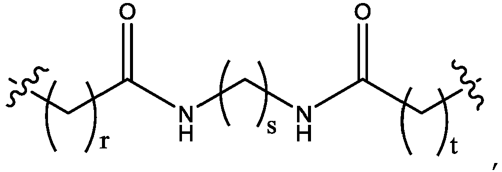 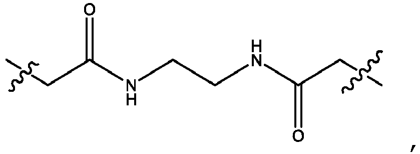 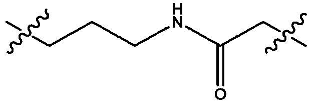 and 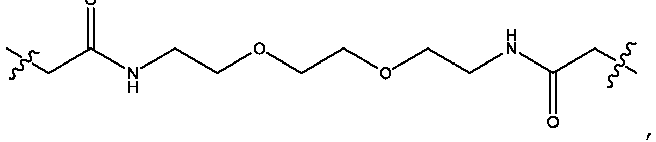 wherein
each of r, s, and t are independently selected from 0, 1, 2, or 3;
w is 1;
X₁, X₂, Y₁ and Y₂ are independently selected from CR₂, O, S, or NR, wherein R is selected from the group consisting of an alkyl group, an acyl group, an aryl group, a cycloalkyl group and a heterocycle group, or R forms an aryl group, a cycloalkyl group or a heterocycle group with another carbon atom within the linker; and
Z₁ and Z₂ is independently selected from O, S or H₂; and optionally
wherein the ligand is selected from the group consisting of: a drug, a metabolite, a peptide, an antigen, a biotin, a hormone, a steroid, a toxin, an antibiotic, an allergen, a protein, a cofactor, a metal, pesticide, insecticide, oligonucleotide, polymer, biopolymer, saccharide, lipid, an electrochemically reactive ligand, parts thereof, derivatives thereof and mixtures thereof.

8. A microarray, comprising a plurality of bead constructs disposed thereon, wherein the bead constructs are as defined in any one of claims 1 to 7.

9. The microarray according to claim 8, wherein said plurality of bead constructs allows identification of the presence of a plurality of target analytes in a sample.

10. A method for manufacturing a microarray, comprising:
depositing a plurality of bead constructs onto a substrate, wherein the bead constructs are as defined in any one of claims 1 to 7.

11. The method according to claim 10, wherein the substrate is selected from the group consisting of polymeric materials, organic materials, inorganic materials, metals, ceramics, plastic, rubber, glass, fibrous materials, graphite or silicon, silicon dioxide, silicon nitride, modified silicon, glass, modified or functionalized glass, inorganic glass, plastics, acrylics, polystyrene, copolymers of styrene, polypropylene, polyethylene, polybutylene, polyurethane, Teflon, polysaccharide, nylon, nitrocellulose, resins, silica, silica-based materials and carbon.

12. A method for identifying the presence of at least one target analyte in a sample, the method comprising:
providing a microarray according to any one of claims 8 to 9;
incubating said microarray with said sample to thereby cause said at least one target analyte, if present in said sample, to bind to at least one ligand molecule, wherein the binding of said at least one target analyte reduces the conformational freedom of the two moieties of a flurophore molecule to each other, to produce a change in an optical signal; and
measuring said change in the optical signal, thereby identifying the presence of said at least one target analyte in said sample.

13. The method according to claim 12, wherein the change in the optical signal is an increase in the level of optical signal after the target analyte binds to the ligand molecule; or
wherein the change in the optical signal is from no optical signal being present before the target analyte binds to the ligand molecule, to the optical signal being present after the target analyte binds to the ligand molecule; or
wherein the change in the optical signal is from a first optical signal being present before the target analyte binds to the ligand molecule, to a second optical signal being present after the target analyte binds to the ligand molecule.

14. A kit for identifying the presence of a target analyte in a sample, the kit comprising:
a microarray according to any one of claims 8 to 9, one or more reagents and instructions for use; and optionally
wherein the one or more reagents are selected from the group consisting of: a fluorescent labeled binding molecule, an antibody, a receptor and an aptamer.

## Patentansprüche

1. Bead-Konstrukt, umfassend:
(a) ein Bead;
(b) ein Fluorophor-Molekül, umfassend zwei Fluorophor-Einheiten, die miteinander durch wenigstens einen internen Linker verbunden sind, der Konformationsfreiheit für die beiden Fluorophor-Einheiten bereitstellt, wobei der interne Linker durch H-Bindungen, Salzbrücken, elektrostatische Wechselwirkung oder wenigstens eine ionische Bindung gebildet wird, und wobei sich ein vom Fluorophor emittiertes optisches Signal ändert, wenn die Konformationsfreiheit der beiden Fluorophor-Einheiten zueinander verringert wird;
(c) ein erstes Linkermolekül, das mit dem Bead und mit einer der beiden Einheiten des Fluorophor-Moleküls verbunden ist;
(d) ein zweites Linkermolekül, das mit den anderen Einheiten des Fluorophor-Moleküls verbunden ist; und
(e) ein Ligandenmolekül, das mit dem zweiten Linker verbunden ist, wobei bei Bindung eines Zielanalyt-Moleküls an das Ligandenmolekül die Konformationsfreiheit der beiden Fluorophor-Einheiten zueinander reduziert wird.

2. Bead-Konstrukt nach Anspruch 1, wobei das Fluorophor-Molekül ausgewählt ist aus der Gruppe bestehend aus:
Chemo- oder Biolumineszenz-Molekülen, aggregationsgequenchten Fluorophoren, aggregationsinduzierten fluoreszierenden Fluorophoren, Black Hole Quenchern gepaart mit regulären Fluorophoren, fluoreszierenden Proteinen, FRET-Farbstoffpaaren, metallverstärkten Fluorophoren, stapelinduzierten Emissions- oder Quench-Fluorophoren, Dexter- und Exciplex-Quenchern; und gegebenenfalls wobei das fluoreszierende Protein ausgewählt ist aus der Gruppe bestehend aus: GFP, RFP, YFP, CFP und Konstrukten davon; und gegebenenfalls
wobei die FRET-Farbstoffpaare ausgewählt sind aus der Gruppe bestehend aus: mOrange2/mCherry, CFP/YFP und FITC/TRITC; und gegebenenfalls
wobei das Chemo- oder Biolumineszenz-Molekül ausgewählt ist aus der Gruppe bestehend aus: Rhodamin, Fluorescein, Coumarin, Acridin, Bodipy, Dansyl, Dapoxy, Hoechst, Cy, Merocyaninen, DAPI, SYTO, Alexa Fluor-Farbstoffen, Pechmann-Farbstoffen, Polymethinen, Biaryl- oder Bi(heteroaryl)-Farbstoffen, Thiophenen, Helicinen, Phenylenen, Xanthenen, Naphthylenen und ihren Derivaten, Isomeren und Konjugaten davon, photochromen Molekülen und aggregationsinduzierten Emittern; und gegebenenfalls
wobei die photochromen Moleküle ausgewählt sind aus der Gruppe bestehend aus: Spiropyranen, Spirooxazinen, Chinonen, Diaryethenen, Azobenzolen und ihren Derivaten, Isomeren und Konjugaten davon; und gegebenenfalls
wobei die aggregationsinduzierten Emitter ausgewählt sind aus der Gruppe bestehend aus: Tetraphenylethen, Hexaphenylsilol, Diphenyldibenzofulven, Silolen und ihren Derivaten, Isomeren und Konjugaten davon; und gegebenenfalls
wobei die metallverstärkten Fluorophore ausgewählt sind aus der Gruppe bestehend aus: Metall-Liganden-Komplexen basierend auf Porphyrinen, Bipyridyl und Phenanthrolinen; und gegebenenfalls
wobei die stapelinduzierten Emissions- oder Quencher-Fluorophore ausgewählt sind aus der Gruppe bestehend aus: Perylen, Pyren, Terylen und ihren Derivaten, Isomeren und Konjugaten davon.

3. Bead-Konstrukt nach Anspruch 2, wobei das Fluorophor-Molekül ein aggregationsinduziertes Fluorophor oder eine Monokomponente oder ein Dikomponenten-Fluorophor ist.

4. Bead-Konstrukt nach einem der vorhergehenden Ansprüche, wobei das Bead einen Durchmesser von 50 nm bis 100 Mikrometer aufweist; und gegebenenfalls wobei das erste Linkermolekül eine Länge im Bereich von 0,2 nm bis 500 nm aufweist.

5. Bead-Konstrukt nach einem der vorhergehenden Ansprüche, wobei das erste Linkermolekül ein organisches Polymer umfasst; und gegebenenfalls wobei das organische Polymer ausgewählt ist aus der Gruppe bestehend aus Polyethylenglykol, Poly(vinylpyrrolidon), Oligo(ethylenglykol), Polyacrylamid, Dextran, Poly-2-methyl-2-oxazolin, Poly(carboxybetainmethacrylat), Poly(L-lysin), Poly(styrolsulfonat), Polyanhydrid, Poly(acrylsäure), Polyethylenimin, Polyglykolsäure und Polycaprolacton.

6. Bead-Konstrukt nach einem der vorhergehenden Ansprüche, wobei das zweite Linkermolekül eine Länge im Bereich von 0,1 nm bis 10 nm aufweist.

7. Bead-Konstrukt nach einem der vorhergehenden Ansprüche, wobei das erste und/oder zweite Linkermolekül ausgewählt ist aus der Gruppe bestehend aus: 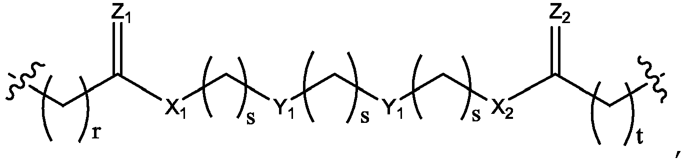 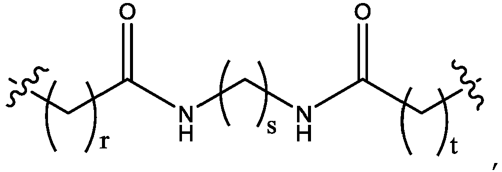 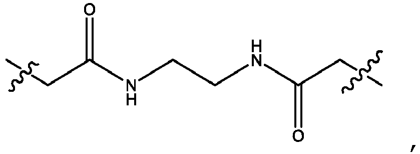 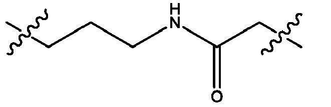 und 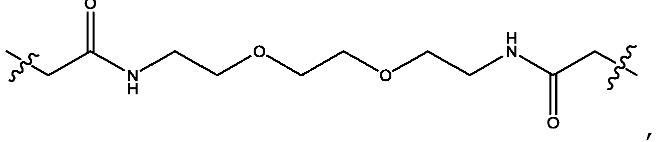 wobei
jedes von r, s und t unabhängig ausgewählt ist aus 0, 1, 2 oder 3;
w 1 ist;
X₁, X₂, Y₁ und Y₂ unabhängig voneinander ausgewählt sind aus CR₂, O, S oder NR, wobei R ausgewählt ist aus der Gruppe bestehend aus einer Alkylgruppe, einer Acylgruppe, einer Arylgruppe, einer Cycloalkylgruppe und einer Heterozyklusgruppe, oder R eine Arylgruppe, eine Cycloalkylgruppe oder eine Heterozyklusgruppe mit einem anderen Kohlenstoffatom innerhalb des Linkers bildet; und
Z₁ und Z₂ unabhängig voneinander ausgewählt sind aus O, S oder H₂; und gegebenenfalls
wobei der Ligand ausgewählt ist aus der Gruppe bestehend aus: einem Wirkstoff, einem Metaboliten, einem Peptid, einem Antigen, einem Biotin, einem Hormon, einem Steroid, einem Toxin, einem Antibiotikum, einem Allergen, einem Protein, einem Cofaktor, einem Metall, Pestizid, Insektizid, Oligonukleotid, Polymer, Biopolymer, Saccharid, Lipid, einem elektrochemisch reaktiven Liganden, Teilen davon, Derivaten davon und Gemischen davon.

8. Mikroarray, umfassend eine Vielzahl von darauf angeordneten Bead-Konstrukten, wobei die Bead-Konstrukte wie in einem der Ansprüche 1 bis 7 definiert sind.

9. Mikroarray nach Anspruch 8, wobei die Vielzahl von Bead-Konstrukten die Identifizierung der Anwesenheit einer Vielzahl von Zielanalyten in einer Probe ermöglicht.

10. Verfahren zur Herstellung eines Mikroarrays, umfassend:
Abscheiden einer Vielzahl von Bead-Konstrukten auf einem Träger, wobei die Bead-Konstrukte wie in einem der Ansprüche 1 bis 7 definiert sind.

11. Verfahren nach Anspruch 10, wobei der Träger ausgewählt ist aus der Gruppe bestehend aus polymeren Materialien, organischen Materialien, anorganischen Materialien, Metallen, Keramik, Kunststoff, Gummi, Glas, faserigen Materialien, Graphit oder Silizium, Siliziumdioxid, Siliziumnitrid, modifiziertem Silizium, Glas, modifiziertem oder funktionalisiertem Glas, anorganischem Glas, Kunststoff, Acryl, Polystyrol, Copolymeren von Styrol, Polypropylen, Polyethylen, Polybutylen, Polyurethan, Teflon, Polysaccharid, Nylon, Nitrocellulose, Harzen, Siliziumdioxid, kieselsäurebasierten Materialien und Kohlenstoff.

12. Verfahren zum Identifizieren der Anwesenheit von wenigstens einem Zielanalyten in einer Probe, wobei das Verfahren umfasst:
Bereitstellen eines Mikroarrays nach einem der Ansprüche 8 bis 9;
Inkubieren des Mikroarrays mit der Probe, um dadurch zu bewirken, dass der wenigstens eine Zielanalyt, falls in der Probe vorhanden, an wenigstens ein Ligandenmolekül bindet, wobei die Bindung des wenigstens einen Zielanalyten die Konformationsfreiheit der beiden Einheiten eines Fluorophor-Moleküls zueinander reduziert, um eine Änderung in einem optischen Signal zu erzeugen; und Messen der Änderung des optischen Signals, wodurch das Vorhandensein des wenigstens einen Zielanalyten in der Probe identifiziert wird.

13. Verfahren nach Anspruch 12, wobei die Änderung des optischen Signals eine Erhöhung des Niveaus des optischen Signals ist, nachdem der Zielanalyt an das Ligandenmolekül gebunden ist; oder
wobei die Änderung des optischen Signals von keinem optischen Signal ausgeht, das vorhanden ist, bevor der Zielanalyt an das Ligandenmolekül bindet, wobei das optische Signal vorhanden ist, nachdem der Zielanalyt an das Ligandenmolekül bindet; oder
wobei die Änderung des optischen Signals von einem ersten optischen Signal ausgeht, das vorhanden ist, bevor der Zielanalyt an das Ligandenmolekül bindet, zu einem zweiten optischen Signal, das vorhanden ist, nachdem der Zielanalyt an das Ligandenmolekül bindet.

14. Kit zum Identifizieren der Anwesenheit eines Zielanalyten in einer Probe, wobei der Kit umfasst:
ein Mikroarray nach einem der Ansprüche 8 bis 9, ein oder mehrere Reagenzien und Gebrauchsanweisungen; und gegebenenfalls
wobei das eine oder die mehreren Reagenzien ausgewählt sind aus der Gruppe bestehend aus: einem fluoreszenzmarkierten Bindungsmolekül, einem Antikörper, einem Rezeptor und einem Aptamer.

## Revendications

1. Construction de bille, comprenant:
(a) une bille;
(b) une molécule de fluorophore, comprenant deux groupements fluorophores liés l'un à l'autre par au moins un lieur interne qui confère une liberté conformationnelle aux deux groupements fluorophores, où le lieur interne est formé par des liaisons H, des ponts de sel, une interaction électrostatique ou au moins une liaison ionique, et où un signal optique émis par le fluorophore change quand la liberté conformationnelle des deux groupements fluorophores l'un par rapport à l'autre est réduite ;
(c) une première molécule de lieur liée à la bille et à l'un des deux groupements de la molécule de fluorophore;
(d) une seconde molécule de lieur liée à l'autre groupement de la molécule de fluorophore; et
(e) une molécule de ligand liée au second lieur, où lors de la liaison d'une molécule d'analyte cible à la molécule de ligand, la liberté conformationnelle des deux groupements fluorophores l'un par rapport à l'autre est réduite.

2. Construction de bille selon la revendication 1, où ladite molécule de fluorophore est choisie dans le groupe consistant en: les molécules chimi- or bioluminescentes, les fluorophores éteints par agrégation, les fluorophores à fluorescence induite par agrégation, les extincteurs à trou noir appariés avec des fluorophores réguliers, les protéines fluorescentes, les paires de colorants FRET, les fluorophores améliorés par des métaux, les fluorophores à émission ou extinction induite par empilement, les extincteurs Dexter et Exciplex; et éventuellement
où la protéine fluorescente est choisie dans le groupe consistant en: GFP, RFP, YFP, CFP et leurs constructions; et éventuellement
où les paires de colorants FRET sont choisies dans le groupe consistant en: mOrange2/mCherry, CFP/YFP et FITC/TRITC; et éventuellement
où la molécule chimi- ou bioluminescente est choisie dans le groupe consistant en: la rhodamine, la fluorescéine, la coumarine, l'acridine, Bodipy, Dansyl, Dapoxy, Hoechst, Cy, les mérocyanines, DAPI, SYTO, les colorants Alexa Fluor, les colorants Pechmann, les polyméthines, les colorants biaryle ou bi(hétéroaryle), les thiophènes, les hélicines, les phénylènes, les xanthènes, les naphtylènes et leurs dérivés, leurs isomères et conjugués, les molécules photochromiques et les émetteurs induits par agrégation ; et éventuellement
où les molécules photochromiques sont choisies dans le groupe consistant en: les spiropyranes, les spirooxazines, les quinones, les diaryéthènes, les azobenzènes, et leurs dérivés, isomères et conjugués; et éventuellement
où les émetteurs induits par agrégation sont choisis dans le groupe consistant en: le tétraphényléthène, l'hexaphénylsilol, le diphényldibenzofulvène, les silols et leurs dérivés, isomères et conjugués; et éventuellement
où les fluorophores améliorés par des métaux sont choisis dans le groupe consistant en: les complexes métal-ligand basés sur des porphyrines, bipyridyle et des phénanthrolines; et éventuellement
où les fluorophores à émission ou extinction induite par empilement sont choisis dans le groupe consistant en: le pérylène, le pyrène, le térylène et leurs dérivés, isomères et conjugués.

3. Construction de bille selon la revendication 2, où la molécule de fluorophore est un fluorophore induit par agrégation, ou un fluorophore monocomposant ou un fluorophore dicomposant.

4. Construction de bille selon l'une quelconque des revendications précédentes, où la bille a un diamètre de 50 nm à 100 micromètres; et éventuellement
où ladite première molécule de lieur a une longueur dans la plage de 0,2 nm à 500 nm.

5. Construction de bille selon l'une quelconque des revendications précédentes, où ladite première molécule de lieur comprend un polymère organique; et éventuellement
où ledit polymère organique est choisi dans le groupe consistant en polyéthylèneglycol, poly(vinylpyrrolidone), oligo(éthylèneglycol), poly-acrylamide, dextrane, poly-2-méthyl-2-oxazoline, poly(méthacrylate de carboxy-bétaïne), poly(L-lysine), poly(styrènesulfonate), polyanhydride, poly(acide acrylique), polyéthylèneimine, polyacide glycolique et polycaprolactone.

6. Construction de bille selon l'une quelconque des revendications précédentes, où ladite seconde molécule de lieur a une longueur dans la plage de 0,1 nm to 10 nm.

7. Construction de bille selon l'une quelconque des revendications précédentes, où ladite première et/ou seconde molécule de lieur est choisie dans le groupe consistant en: 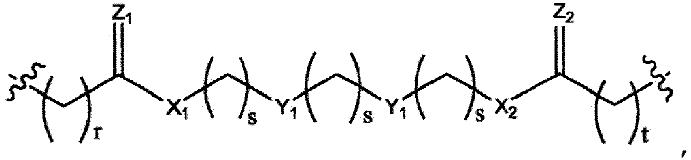 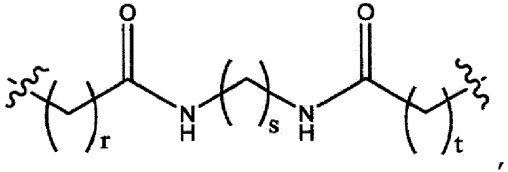 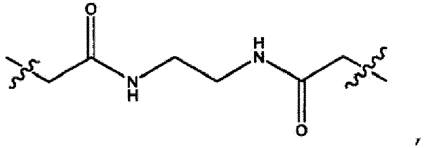 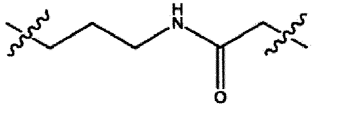 et 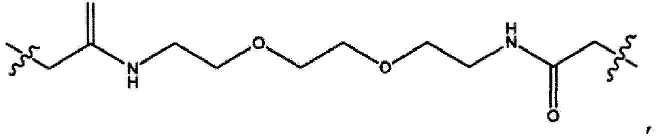 où
r, s et t sont choisis chacun indépendamment parmi 0, 1, 2 et 3;
w est 1 ;
X₁, X₂, Y₁ et Y₂ sont choisis indépendamment parmi CR₂, O, S et NR, où R est choisi dans le groupe consistant en un groupe alkyle, un groupe acyle, un groupe aryle, un groupe cycloalkyle et un groupe hétérocycle, ou R forme un groupe aryle, un groupe cycloalkyle ou un groupe hétérocycle avec un autre atome de carbone dans le lieur ; et
Z₁ et Z₂ sont choisis indépendamment parmi O, S et H₂;
et éventuellement
où le ligand est choisi dans le groupe consistant en: un médicament, un métabolite, un peptide, un antigène, une biotine, une hormone, un stéroïde, une toxine, un antibiotique, un allergène, une protéine, un cofacteur, un métal, un pesticide, un insecticide, un oligonucléotide, un polymère, un biopolymère, un saccharide, un lipide, un ligand réactif électrochimiquement, des parties de ceux-ci, des dérivés de ceux-ci et des mélanges de ceux-ci.

8. Micropuce, comprenant une pluralité de constructions de billes disposées sur elle, où les constructions de billes sont comme défini dans l'une quelconque des revendications 1 à 7.

9. Micropuce selon la revendication 8, où ladite pluralité de constructions de billes permet l'identification de la présence d'une pluralité d'analytes cibles dans un échantillon.

10. Procédé pour fabriquer une micropuce, comprenant:
le dépôt d'une pluralité de constructions de billes sur un substrat, où les constructions de billes sont comme défini dans l'une quelconque des revendications 1 à 7.

11. Procédé selon la revendication 10, où le substrat est choisi dans le groupe consistant en les matériaux polymériques, les matériaux organiques, les matériaux inorganiques, les métaux, les céramiques, les matières plastiques, le caoutchouc, le verre, les matériaux fibreux, le graphite ou le silicium, le dioxyde de silicium, le nitrure de silicium, le silicium modifié, le verre, le verre modifié ou fonctionnalisé, le verre inorganique, les matières plastiques, les matières acryliques, le polystyrène, les copolymères de styrène, le polypropylène, le polyéthylène, le polybutylène, le poluyuréthane, le Téflon, un polysaccharide, le nylon, la nitrocellulose, les résines, la silice, les matériaux à base de silice et le carbone.

12. Procédé pour identifier la présence d'au moins un analyte cible dans un échantillon, le procédé comprenant :
la fourniture d'une micropuce selon l'une quelconque des revendications 8 à 9 ;
l'incubation de ladite micropuce avec ledit échantillon pour amener ledit au moins un analyte cible, s'il est présent dans ledit échantillon, à se lier à au moins une molécule de ligand, où la liaison dudit au moins un analyte cible réduit la liberté conformationnelle des deux groupements d'une molécule de fluorophore l'un par rapport à l'autre, pour produire un changement dans un signal optique ; et
la mesure dudit changement dans le signal optique, identifiant ainsi la présence dudit au moins un analyte cible dans ledit échantillon.

13. Procédé selon la revendication 12, où le changement dans le signal optique est un accroissement dans le niveau de signal optique après la liaison de l'analyte cible à la molécule de ligand ; ou bien
où le changement dans le signal optique est de l'absence de signal optique avant la liaison de l'analyte cible à la molécule de ligand à la présence de signal optique après la liaison de l'analyte cible à la molécule de ligand ; ou bien
ou le changement dans le signal optique est de la présence d'un premier signal optique avant la liaison de l'analyte cible à la molécule de ligand à la présence d'un second signal optique après la liaison de l'analyte cible à la molécule de ligand.

14. Kit pour identifier la présence d'un analyte cible dans un échantillon, le kit comprenant :
une micropuce selon l'une quelconque des revendications 8 à 9, un ou plusieurs réactifs et des instructions pour l'utilisation ; et éventuellement
où les un ou plusieurs réactifs sont choisis dans le groupe consistant en : une molécule de liaison marquée par fluorescence, un anticorps, un récepteur et un aptamère.
